# EUROPEAN PATENT APPLICATION

(11) **EP 3 207 868 A1**
(43) Date of publication of application: **23.08.2017**
(21) Application number: 16156510.6
(22) Date of filing: 19.02.2016
(51) Int. Cl.: A61B 5/08, A61B 5/16

(54) **METHOD AND APPARATUS FOR IDENTIFYING A TRANSITORY EMOTIONAL STATE OF A LIVING MAMMAL**

(71) Applicant: Patonomics AB, 414 64 Göteborg (SE)
(72) Inventor: MITRA, Kalyan, 414 64 GÖTEBORG (SE)
(74) Representative: Awapatent AB

(57) **Abstract**

A method and apparatus for automated identification of at least one transitory emotional state, such as fear, happiness, disgust, etc, of a living mammal are disclosed. At least one sensor is mounted on or in the vicinity of the living mammal, said sensor being configured to detect at least one metabolite molecule(s), and preferably at least one volatile organic compound(s), emanated by the living mammal. Continuously and in real-time the amount of at least one of the metabolite molecule(s) is then determined with the sensor over a period of time. The determined amount of the metabolite molecule(s) is further analyzed to establish whether the amount or the increase rate of the metabolite molecule(s) exceeds at least one predetermined criterion, and when such a predetermined criterion has been exceeded, identifying that a transitory emotional state associated with the metabolite molecule(s) is present in the living mammal.

## Description

### Field of the invention

The present invention generally relates to a method and apparatus for automated identification of one or more transitory emotional state(s).

### Background of the invention

Living mammals, such as humans but also many animals, have many transitory emotional states occurring from time to time, such as anger, fear, laughter, happiness, etc. Recognizing such transitory emotional states is of great importance in many situations, such as in evaluating the impact and effect of real-time emotional well being in multiple situations like personal, professional and commercial settings, and ability of activating and offering targeted real time response mechanism matching with transitory emotional states, emotional appeal assessment in new product design (physical product design or digital product design including content creations for marketing and advertising industry & movie or film Industry), monitoring and intervening high performance training, when questioning people in judicial situations and the like, when evaluating and enhancing medical therapy and the like. However, identification of transitory emotional states is today only made subjectively by human individuals.

There have been some attempts to automate the recognition of some mental states, as e.g. disclosed in WO 2915/03952 and WO 2014/145228. Here, an attempt is made to recognize facial expressions from image data, and to connect this to various mental states. However, many transitory mental states are difficult to identify and distinguish from each other based on image data, and there is still a need for improved ways of identification of transitory emotional states in a simpler and more efficient and reliable way.

Pioneering work on human and mammalian olfaction is done by Dr. Noam Sobel since 1997 but most prominent studies are e.g., "Blind smell: Brain activation induced by an undetected air-borne chemical" by Noam Sobel et al, Brain, 1999, vol. 122, No. 2, 209-217, confirms that there are some molecules that mammals cannot consciously smell, but brain get activated anyway. Following work has pin pointed that mammalian bio-fluids contains emotional signals. Over the decades many of Noam Sobel 's work is remarkable. And here are some important and representative publications from him. "Sniffing longer rather than stronger to maintain olfactory detection threshold" by Noam Sobel et al, Chemical Senses, 2000, vol. 25, No. 1, 1-8, "Rapid olfactory processing implicates subcortical control of an olfactomotor system" by Bradley N.Johnson et al, Journal of Neurophysiology, 2003, vol. 90, No. 2, 1084-1094, "The influence of odorants on respiratory patterns in sleep" by Anat Arzi et al, Chemical Senses, 2009, vol. 35, No. 1, 31-40, "Spatial perception: Time tells where a smell comes from" by Anat Arzi et al, Current Biology, 2010, vol. 20, No. 13, 563-564, "Sniffing enables communication and environmental control for the severely disabled" by Anton D.Plotkin et al, Proceedings of the National Academy of Sciences of the United States of America, 2010, vol. 107, No. 32, 14413-14418, "Human Tears Contain a Chemosignal" by Shani Gelstein et al, Science, 2011, vol. 331, No. 6014, 226-230, "Humans can learn new information during sleep" by Anat Arzi et al, Nature Neuroscience, 2012, vol. 15, No. 10, 1460-1465, "An Assay for Human Chemosignals" by Idan Frumin et al, Methods in Molecular Biology, 2013, vol. 1068, 373-394, "Mirror sniffing: Humans mimic olfactory sampling behavior" by Anat Arzi et al, Chemical Senses, 2014, vol. 39, No. 4, Article No. bjt113, 277-281, "A Mechanistic Link between Olfaction and Autism Spectrum Disorder" by Liron Rozenkrantz et al, 2015, vol. 25, No. 14, 1904-1910, "A social chemosignaling function for human handshaking" by Idan Frumin et al, eLife, 2015, vol. 2015, No. 4, Article No. e05154, 16p.

Further, some studies have been made on olfactory communication. Such studies are e.g. "Chemical Communication of Fear: A case of Male-Female Asymmetry" by J. de Groot and G. Semin, Journal of Experimental Psychology, 2014, vol. 143, No. 4, 1515-1525, "I Can See, Hear, and Smell Your Fear: Comparing Olfactory and Audiovisual Media in Fear Communication", by J. de Groot and G. Semin, Journal of Experimental Psychology, 2014, vol. 143, No. 2, 825-834, "A Sniff of Happiness" by J. de Groot et al, Psychological Science 2015, vol. 26(6), 684-700, "Chemosignals Communicate Human Emotions" by J. de Groot et al, Psychological Science 2012, vol. 23(11), 1417-1424, "The chemical bases of human society" by G. Semin and J. de Groot, Trends in Cognitive Sciences, vol. 17, no. 9, Sept. 2013, and "Rapid Stress System Drives Chemicla Transfer of Fear from Sender to Receiver" by J. de Groot et al, Plos One, Feb. 27, 2015. In these studies, sweat emanating from people in various emotional states have been evaluated by other people, and it has been found that the odor from the sweat can communicate certain emotional states, such as fear, to the receivers. However, even though highly interesting from the point of understanding the full width of interpersonal communication occurring between individuals, the practical use of these studies is limited, and the findings cannot be used for automated proceedings.

The state of art as of Aug 2014 is captured by "No one has yet found a molecule in human sweat that corresponds to our level of anxiety. Several labs have tried to measure the effects of sniffing someone else's fear-inspired body odor" from "ASK ANYTHING: CAN HUMANS SMELL FEAR? SHORT ANSWER: IT'S UP FOR DEBATE" By Daniel Engber Posted July 21, 2014 http://www.popsci.com/article/science/ask-anything-can-humans-smell-fear

The following work has establish that physical stress has increase some breath molecules - "Dynamic profiles of volatile organic compounds in exhaled breath as determined by a coupled PTR-MS/GC-MS study" by J. King et al, Physiological Measurement, No. 31 (2010), 1169-1184. But no known volatile molecule has been identified for positive and negative emotional states that emanates from breath or from skins.

Still further, in the paper "Cinema Data Mining: The Smell of Fear" by Wicker et al, Proceedings of the 21st ACM SIGKDD International Conference on Knowledge Discovery and Data Mining, p. 1295-1304, a study is reported in which air in a cinema was studied. Even though this study indicates that there are some correlation between some ions in the air of the cinema and the movies being shown, these findings are not useable to determine transitory emotional states in individuals in a simple and reliable way, or in any automated and real-time fashion.

Thus, none of the above-discussed studies can be used for identification of transitory emotional state(s) in individual living mammals in a useful way. Consequently, there is still a need for improved ways of identification of transitory emotional states in a simpler and more efficient and reliable way.

There is therefore still a need for improvements in automated methods and apparatuses for identification of transitory emotional states.

### Summary of the invention

It is a general object of the present invention to alleviate the above-discussed problems, and at least partly satisfying the above-discussed needs.

This object is fulfilled by a method and an apparatus for identification of at least one transitory emotional states of a living mammal in accordance with the appended claims.

According to a first aspect of the present invention, there is provided a method for automated identification of at least one transitory emotional state of a living mammal, comprising the steps:
mounting at least one sensor on or in the vicinity of said living mammal, said sensor being configured to detect at least one metabolite molecule(s), and preferably volatile organic compound(s) or volatile metabolite molecule(s), emanated by the living mammal;
determining continuously and in real-time the amount of at least one of said metabolite molecule(s) with said sensor over a period of time;
analyzing said determined amount of said at least one metabolite molecule(s) to establish whether the amount or the increase rate of said at least one metabolite molecule(s) exceeds at least one predetermined criterion, and when such a predetermined criterion has been exceeded, identifying that a transitory emotional state associated with said at least one metabolite molecule(s) is present in the living mammal.

It has been recognized by the present inventor that certain metabolite molecules are biomarkers clearly related to certain distinguishable transitory emotional states, and that this can be used for fast and reliable automated identification of such transitory emotional states. Hereby, it becomes possible to use automated identification of one or several transitory emotional states for a variety of purposes, as will be discussed in more detail in the following, and in a variety of various fields, such as in product design (physical or digital), individual well being, medical therapy, nursing, psychological therapy, marketing, advertising, training and education, legal work, sports psychology, etc.

The identification of the transitory emotional state(s) can be made subsequent to the measurement, but is preferably made in real-time, and preferably on a continuous basis over a period of time.

It has been found that the disclosed method and apparatus is particularly suitable for identification of the transitory emotional states fear, stress, anger, sadness, disgust, surprise, laughter and happiness.

The sensor is preferably arranged as a wearable sensor, to be worn by the living mammal.

The at least one sensor preferably comprises a sensor configured to be mounted in or in the vicinity of a mouth or nose of the living mammal, said sensor being configured to detect at least one metabolite molecule(s) emitted by the living mammal in its breath. Hereby, airborne metabolite molecules can easily be detected and quantified.

In one embodiment, the sensor may be arranged to be positioned within the mouth, such as in a tubular sensor arrangement, held between the lips, or as a small sensor module, placed on the skin inside the mouth, or as a sensor placed partly or fully within a nostril. Such nostril sensors can e.g. be arranged as a clip, with one jaw to be placed inside the nostril and one jaw to be placed outside the nostril. Examples of such wearable sensor arrangements, to be placed within a nostril or to be added to nose inserts are per se known from e.g. WO 2015/008047, US2006085027(A1), US8403954(B2), AU2013209347(B2), US2007062538, said documents hereby being incorporated in their entirety by reference.

However, it is also feasible to arrange the sensor at some distance from the mouth and nose, such as being arranged within a few mm or cm away. For example, the sensor may be worn in the same way as a headset microphone or the like, i.e. by being held by a holding structure positioned over the head or over one or both ears, or over the shoulder, and with an arm, holding the sensor, being arranged to extend to a position relatively close to the mouth or nose. In a corresponding way, the sensor may be arranged in a hat or other clothing, such as in cloth buttons, in jewelry, such as nose jewelry or other clothing accessories. It is also possible to arrange the sensor on the frame of glasses, around the neck like a necklace, around feet's like in shoes and the like.

When measuring on breath, the time period for determining continuously the amount of at least one of said, preferably volatile, metabolite molecule(s) preferably extends over at least one, and preferably a plurality, of breathing cycles.

Alternatively or additionally, metabolite molecule(s) emanating through the skin of the living mammal may be detected and quantified. Thus, the at least one sensor may comprise a sensor configured to be mounted on the skin of the living mammal, said sensor being configured to detect at least one metabolite molecule(s) emitted through the skin of the living mammal. This type of sensors can be adhered to the skin on various places on the body, as is per se known, It may also be worn around the wrist, e.g. in a bracelet, or as an integral part of an electronic watch. Such sensor arrangements are also per se known, e.g. from US 2015/0289790 and US 8823524, both said document hereby being incorporated in their entirety by reference.

The at least one, preferably volatile, metabolite molecule(s) preferably comprises at least one of the ammonia, acetic acid, acrolein (C₃H₄O), and molecule(s) that produce ion fragments C₃H₇⁺ and C₃H₅⁺.

The at least one predetermined criterion used to evaluate the measurement data can be of various types. Identification may be determined based on the determined amount (or concentration) of the metabolite molecule(s), in absolute measures or as a relative measure in relation to calibration for each living mammal's individual concentration in steady neutral state.

The predetermined criterion to identify the transitory emotional state(s) may here be threshold values, determined either statistically, as an average from many measurements on other persons, or in comparison with previous measurements on the same person. For example, when measuring over time, an average value may be determined, and the criterion may be that the measured amount exceeds the average value with a certain amount, such as 20%, 50%, 100% or even more, or the like. In the same way, the criterion may related to increase rate, such as when the amount increases with a certain amount, such as 20%, 50%, 100% or even more, or the like, within a certain time period, such as within 2 seconds, 5 seconds, 10 seconds, 50 seconds or more.

The disclosed method and apparatus for identifying one or several transitory emotional state(s) in a human or an animal may be used with great advantage in many different fields, some of which will be discussed briefly in the following. However, this list is not exhaustive, and many other uses may also be contemplated.

Thus, the identified transitory emotional state may be used in at least one of:
- Overcoming communication difficulties and help in communication. By determining, in real-time, the emotional state of a person, communication is improved and enriched, which is e.g. highly advantageous when communicating with persons having trouble expressing themselves verbally, due to mental or physical illness, lack of verbal skills, due to traumas, accidents, stroke, birth defects, and the like. It may also be used for improved communication with animals.
- Monitoring post surgery recovery. By automated and preferably continuous monitoring of the emotional state of patients, complications and the like can easily be discovered at an early stage, leading to less pain for the patients, and improved therapeutic results.
- Monitoring sleep quality. By monitoring the transitory emotional states, the sleep quality may be monitored, which also makes it possible to identify reasons behind poor sleep quality, and to aid in the finding of measures to improve the sleep quality.
- Monitoring pattern in sleep disorder. In a similar way, it also becomes possible to identify patterns for persons with sleep disorder, and thereby aiding in finding solutions to overcome this.
- Testing of commercial audio and/or video media. By monitoring the emotional reactions from test persons when watching new advertising and/or movie materials and the like, it becomes possible to obtain immediate and objective feedback on this, which is highly advantageous.
- Testing emotional expected appeal in new product design (for either physical, digital products or audio video media).
- Reliability testing of suspects. By providing the additional dimension of the emotional state, the reliability of an individual's spoken communication and actions can be determined.
- Reliability testing on accused and witness during judicial trials. When questioning witnesses suspects and the like, objective identification of transitory emotional states of the person being questions enrich the information provided, and provide strong indications of the reliability of what is being communicated orally.
- Self performance monitoring. Monitoring the objectively determined emotional state of oneself can be useful in many ways, such as for improving training results, identification of activities and the like which are likely to drain your motivation, attention focus and joy, for improved mental training, etc.
- Professional performance monitoring and real-time feedback for performance improvement. It may also be used by professionals, e.g. for finding candidates for a new job position, for evaluating individuals and teams in a company, for improving training of employees, professionals, pilots, athletes, etc.
- Customizing mobile content delivery. It may also be useful for improving and customizing mobile content, by evaluating proposals objectively, based on the emotional reactions that are obtained.
- Customizing physical and/or emotional environment. It may also be useful for improving and customizing physical environment - e.g. in respect of temperature, pressure, air flow, humidity, controlling doors and windows operation, selective media presentation or combinations thereof - by evaluating proposals objectively, based on the emotional reactions that are obtained.
- Customizing robot, and robotic control. It may also be useful for improving and customizing robot or robotic pets or robotic pals or automation response - by evaluating proposals objectively, based on the emotional reactions that are obtained.
- Aid in learning and memory training. It may also be useful for improving and customizing learning and memory training by injecting right quantity positive emotional and motivational volatile biomarkers and/or expelling or destroying the negative emotional and motivational volatile biomarkers to the learning environment.
- Aid in commercial environments. It may also be useful for improving and customizing consumer environment by injecting right quantity positive emotional and motivational volatile biomarkers and/or expelling or destroying the negative emotional and motivational volatile biomarkers from the closed or close-like environments at different kind commercial spaces, like, school, shopping places, shopping malls, hospitals, offices, movie theaters, stadiums, etc.
- Aid in transportation environments. It may also be useful for improving and customizing transportation environment by injecting right quantity positive emotional and motivational volatile biomarkers and/or expelling or destroying the negative emotional and motivational volatile biomarkers from the closed or close-like environments at different including all transportation and spacecrafts environments.
- Monitoring progress of psychological therapy. In psychology, the mental state is of enormous importance, and objective information and feedback on the emotional state(s) is very useful for improving the therapy and for obtaining better treatment results.
- Diagnosing mental disease. It may also be useful for identification of mental disorders and mental diseases.
- Diagnosing state of dementia. In the same way, it may be used for diagnosing dementia already at an early stage.
- Diagnosing depression. In the same way, it may be used for diagnosing depression already at an early stage.
- Quality ensuring consumer research. It may also be used as a tool for ensuring quality and reliability of consumer research.
- Testing during neuromarketing. By "neuromarketing" is here meant a field of marketing research that studies consumers' sensorimotor, cognitive, and affective response to marketing stimuli.
- Emotional status tracking in vulnerable subject, e.g. during bullying. By monitoring the emotional states continuously or over certain time periods, it becomes possible to identify e.g. bullying of kids, elderly and the like. For example, it can hereby easily be determined if a kid experiences fear at certain times during the school day, such as during the breaks, which is a clear indication of bullying or the like.
- Emotional tracking of patients under anesthesia or coma. It now also becomes possible to monitor the emotional state of persons being incapacitated, and where there is no possibility of verbal communication, such as during anesthesia or patients in coma.
- Promoting physical training. Monitoring of the emotional state during physical training is a powerful tool in establishing improvements in training schemes and the like, and thereby improves both the experience and result of the training.
- Aiding emotional communication, in particular with patients suffering from dementia, mental retardness, stroke and brain damage, and with animals. By objective identification and monitoring of the mental state of a person or an animal, it becomes possible to enhance the emotional communication, e.g. by obtaining emotional feedback on various actions etc. Hereby, a better quality of life can be obtained.
- Aiding in Human Resource selection. It is also highly useful as an aid in human resources, e.g. when hiring new employees.
- Monitoring and aiding in emotionally connecting or understanding domestic pets by the owners or veterinary professionals.
- Monitoring and aiding with commercial animals for commercial objective like mammals used in pharmaceutical research, in medical treatment by veterinary professionals, performance measurement of horses in sports or betting industry, domestication training of mammals, better quality commercial products from commercial animals like in meat and milk production etc.
- Communicating emotional status over telephone, mobile, VOIP network from a sender to a receiver and vice versa.

The method and apparatus of the present invention is of particular interest for identification of positive emotions like happiness and in laughing situations, and wherein the metabolite molecule(s) are one or a few molecules that produce of fragments like, C₃H₇ +, and C₃H₅⁺.

The method and apparatus of the present invention is of particular interest for identification of negative emotions like disgust and fear situations, and wherein the metabolite molecule(s) are one or more of the molecules: acetic acid, ammonia, acrolein and molecules which have molecular formula C₃H₄O, and in particular at least one of acetic acid and ammonia.

According to another aspect of the invention, there is provided an apparatus for automated identification of at least one transitory emotional state of a living mammal, comprising:
at least one sensor configured to be mounted on or in the vicinity of said living mammal, said sensor being configured to detect in real-time at least one metabolite molecule(s), and preferably and preferably volatile organic compound(s) or volatile metabolite molecule(s), emitted by the living mammal;
a memory to store data related to the amount of at least one of said metabolite molecule(s) during continuous measurement with said sensor over a period of time;
an analyzer programmed to analyze said determined amount of said at least one metabolite molecule(s) to establish whether the amount or the increase rate of said at least one metabolite molecule(s) exceeds at least one predetermined criterion, and when such a predetermined criterion has been exceeded, identifying that a transitory emotional state associated with said at least one metabolite molecule(s) is present in the living mammal.

Hereby, similar advantages and preferred features and embodiments as discussed above in relation to the firstly discussed aspect are obtainable.

The sensor is preferably arranged remote from said analyzer, the analyzer and the sensor being connected by a wired or wireless connection.

The apparatus may further advantageously comprise a feedback provider providing a visual or audial indication of the identified transitory emotional state. Thus, the identified emotional state may be visualized in real-time on a display, signaled by lights in different colors, presented by various sounds, or the like.

The at least one sensor may comprise a sensor configured to be mounted in or in the vicinity of a mouth or nose of the living mammal, said sensor being configured to detect at least one metabolite molecule(s) emitted by the living mammal in its breath, said sensor preferably being attachable to a nostril of the living mammal, and e.g. comprising a clip-on sensor comprising a pair of jaws to clip the module partially within said nostril.

The apparatus may further preferably comprise at least one, and preferably two or more, of: an output device, such as a display, an alarm, or the like; an interface for communicating data, such as measurement data and/or data about determined transitory emotional state(s) to an external device, and preferably through wireless communication; and an input device to provide control data, setting data, reconfiguration data or the like to the apparatus.

According to still another aspect of the present invention, there is provided a use of one or several molecules that produce of fragments like, C₃H₇⁺, and C₃H₅⁺.

Hereby, similar properties and advantages as discussed above in relation to the other aspects of the invention are obtainable.

These and other aspects of the inventive concept will be apparent from and elicited with reference to the embodiments described hereinafter.

### Brief description of the drawings

By way of example embodiments of the invention will now be described with reference to the accompanying drawings in which:
Fig. 1 schematically illustrates an embodiment of an apparatus according to one embodiment of the invention; and
Figs. 2-5 are exemplary diagrams showing the variation of certain metabolite molecules measured in the breath of test persons during experiments.

### Description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention. Further, even though the following detailed description focuses on monitoring of human beings, it is to be appreciated by the skilled reader that it is equally applicable to animals and in particular commercial and/or domestic animals or zoo animals, such as horses, dogs, cows, cats, primates, rats, mice and the like. It is noticed that commercial and domesticated animals smell out emotions in other living mammals and react accordingly.

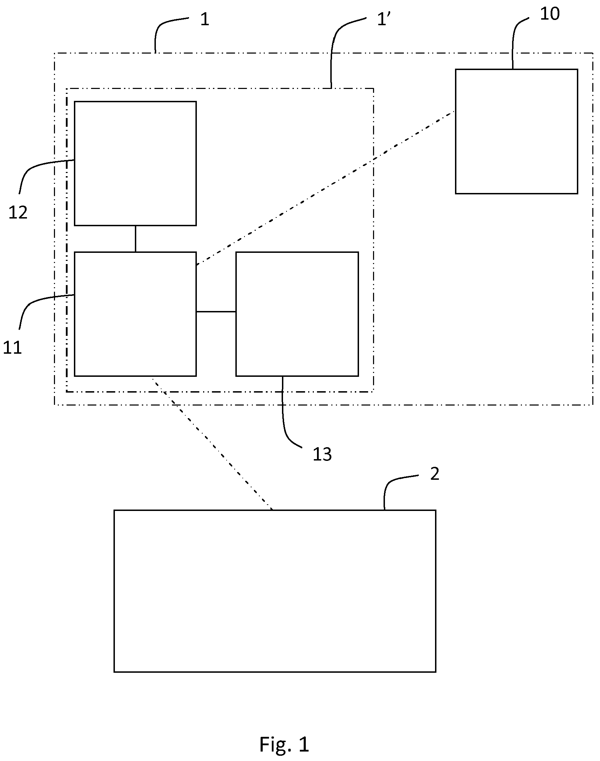

Referring to Fig. 1, an apparatus 1 according to embodiments of the invention comprise at least one sensor 10, for detection and quantification of one or more metabolite molecule(s), an analyzer 11, a memory 12 and optionally an output device 13. All these parts may be integrated as a single unit, as indicated by dashed line 1 in Fig. 1. However, alternatively, one or more of the parts may be arranged as separate parts, connected to the other parts by means of wired or wireless connections. For example, the sensor may be arranged as a separate part, arranged to communicate with the analyzer via e.g. Bluetooth. Such an alternative arrangement is schematically illustrated by dashed line 1' in Fig. 1.

As discussed in the foregoing, the sensor 10 may be configured to measure one or several metabolite molecules emanating from the body, either in the breath or through the skin. Sensors structured to be placed in the vicinity of the mouth or nose and on the skin are per se well known, and need not be further discussed. Further, measurement methods for determining presence and quantity of various metabolite molecules are also per se known. For example, such measurement may involve Proton Transfer Reaction Mass Spectrometry (PTR-MS), Selected Ion Flow Tube Mass Spectrometry (SIFT-MS) and Ion Mobility Spectrometry (IMS). These and other suitable measurement methods are per se known, and are e.g. further discussed in "Dynamic profiles of volatile organic compounds in exhaled breath as determined by a coupled PTR-MS/GC-MS study" by J. King et al, Physiological Measurement, No. 31 (2010), 1169-1184, "Ammonia in breath and emitted from skin" by F. Schmidt et al, Journal of Breath Research, No. 7 (2013), and "Breath Analysis as a Potential and Non-Invasive Frontier in Disease Diagnosis: An Overview" by J. Pereira et al, Metabolites, No. 5, 2014, all of said documents hereby being incorporated in their entirety by reference. There are multiple research labs and companies that offer mico and nano sensors that have capability to measure specific gasses or volatile compounds in real time or near real time. The sensor for wearable emotion sensing may preferably use portable gas sensors. Such sensors are e.g. commercially available from Sensirion and Sensotran, and there are also known gas sensors technologies that use infrared or near infrared for real time gas sensing. There is at present a lots of research related to miniaturization of real time gas sensors, and such gas sensors, when commercially available, can also be use for implementing the invention.

The sensor 10 may be arranged as a separate part, and is preferably communicating with the analyzer over a wireless connection, such as by Bluetooth. However, the sensor may also have a wired connection to the analyzer, or even form an integrated unit together with the analyzer. Preferably, the sensor is arranged as a wearable module.

The analyzer 11 is a computer/processor controlled part, arranged to perform the above-discussed method for identification of transitory emotional state(s) by executing a software code. However, the method may also be partly or fully implemented by hardware.

The analyzer is configured to identify one or several transitory emotional state(s) by executing the following steps:
- Reception of measurement data from the sensor or from the memory 12;
- Analyzing the measurement data related to the amount of the at least one metabolite molecule(s) to establish whether the amount or the increase rate of said at least one metabolite molecule(s) exceeds at least one predetermined criterion;
- Determining, when such a predetermined criterion has been exceeded, that a transitory emotional state associated with this at least one metabolite molecule(s) is present in the living mammal; and
- Forwarding, optionally, the result of the analysis and/or the determination to the output device 13, and/or to the memory 12 for storage.

The memory 12 may be any type of memory device arranged to store data in a retrievable fashion.

The output device 13 may take various forms. It may be a display, for presenting information in relation to the determined emotional/mental state(s), in writing, by showing images, showing concentration pots over time, or the like. It may also be one or several lights, e.g. lights of various colors, which are illuminated, upon identification of a particular emotional/mental state. For example, a green light may be active when happiness has been detected, a red light may be active when fear or anger has been detected, and a yellow light may be active when no emotional state has been identified. The output device may also be a loudspeaker, emitting signals indicative of the identified mental state(s). Many other type of output devices may also be used, as would be apparent for the skilled reader. The aggregate review of emotions can be visualized and viewed for any time period, such as specific seconds, specific hours, specific days, weeks, months, year or years. Various combinations of these and other output devices may also be used.

Data regarding e.g. identified emotional/mental state(s), measurement data, etc may also be forwarded to separate external devices 2, such as to an external tablet, general purpose computer, electronic watch, smart phone, etc. This transfer of data may be provided through wired connections, but preferably occurs through wireless communication, such as by conventional TCP/IP communication. To this end, both the analyzer and the separate external device may preferably be provided with capability and access to communicate via the Internet.

The analyzer may also send an alarm signal or the like to an external device 2 when certain mental state(s) has been identified. For example, an alarm may be sent to a caregiver or nursing personnel when a patient experiences fear.

In such a case, when issuing an alarm, the apparatus may further communicate the present position of the apparatus together with the alarm. Such position data may be entered manually to the apparatus, or be retrieved from other sources. However, the apparatus may also comprise a Global Navigation Satellite System (GNSS) receiver, such as a GPS receiver, to obtain such positioning data.

The apparatus may further comprise an input device (not shown), such as a keyboard or the like, enabling the user to modify the performance of the device. For example, this may be used to vary the identification criteria used by the device, so that the sensitivity of the apparatus is controlled to a desired level. It may also be used to control the output format, and the like. Additionally or alternatively, such control of the apparatus may also be obtained through one or several external devices 2, the external device(s) thereby functioning as a remote control for the apparatus.

### Experimental results.

In a series of experiments, the breath of test persons was continuously analyzed over a period of time. During the entire measurement, the test persons were watching a film containing sequences expected to arouse various emotions in the viewer, such as happiness, laughter, fear, disgust, etc. The test persons were also all interviewed afterwards, to confirm about what emotions they had experienced over the measurement period.

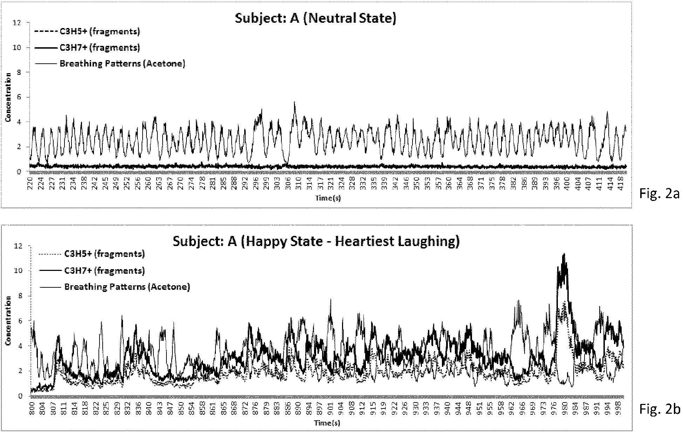

Representative measurement results of some chosen metabolite molecules for some of the test persons are shown in the diagrams of Figs. 2-5.

In figs 2a and 2b, correlation between C₃H₅⁺ and C₃H₇⁺ and happiness are illustrated. Fig 2a illustrate a diagram showing these ions together with acetone in an example individual A in a neutral state, whereas fig 2b illustrate these ions together with acetone for the same individual A, when being in a happy state (laughing heartily). As is clearly determinable from these figures, there is a strong correlation between these ions and the emotional state. In the happy state, both the quantity of C₃H₅⁺ and C₃H₇⁺ are significantly and dramatically increased, compared to the neutral state, while the level of acetone remains essentially the same, and the same pattern is found also for other major breath metabolites, which also remain essentially unaffected. Further, the volatility of both these ions increases dramatically in the happy state, and there is an increased variation between highest and lowest levels.

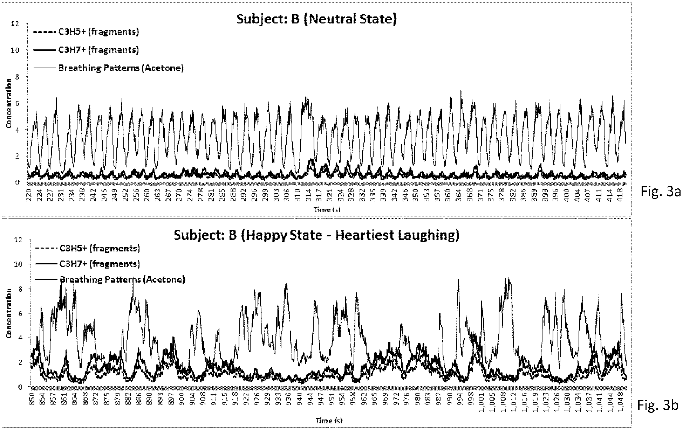

The same pattern is seen in Figs. 3a and 3b, showing the correlation between C₃H₅⁺ and C₃H₇⁺ together with acetone in another example individual B in a neutral state and a happy state (laughing heartily), respectively.

Even though Figs 2 and 3 only show the measurement for two specific individuals, the same pattern has been found and confirmed in numerous other measurements on the same and other individuals.

It has been concluded that happiness can be determined based on measurement of these ions, by measuring the quantities of either or both of these ions, in absolute terms, by measuring the quantities in relation to a calibration value, such as the level of acetone, or by trend analysis over time, etc.

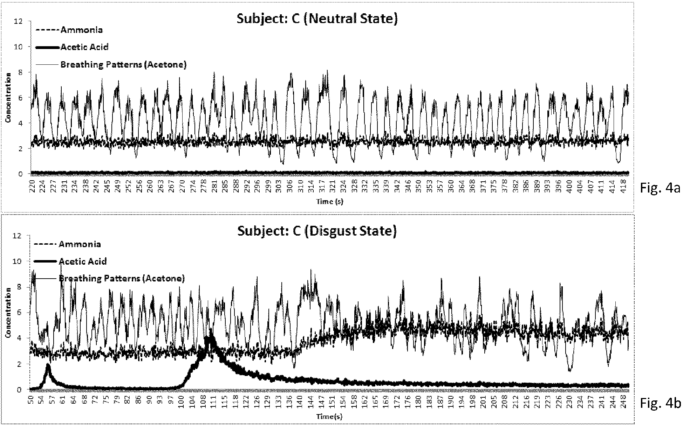

In figs 4a and 4b, correlation between ammonia and acetic acid and disgust are illustrated. Fig 4a illustrate a diagram showing these molecules together with acetone in an example individual C in a neutral state, whereas fig 4b illustrate these molecules together with acetone for the same individual C, when being in a state of disgust. As is clearly determinable from these figures, there is a strong correlation between these molecules and the emotional state. In the disgust state, the quantity of ammonia is raised dramatically, to a relatively stable level being significantly higher than the stable layer of the neutral state. The quantity of ammonia is raised steadily during a transition period of a number of breathing cycles when the disgust state has been entered, and is thereafter stable on this new, higher, level, and slowly falls back towards a lower level again.

The level of acetic acid shows another type of variation. As soon as the disgust state is entered, there is an almost immediate and highly dramatic increase in the quantity of acetic acid. This high level then very soon and quite rapidly starts to return to the normal level.

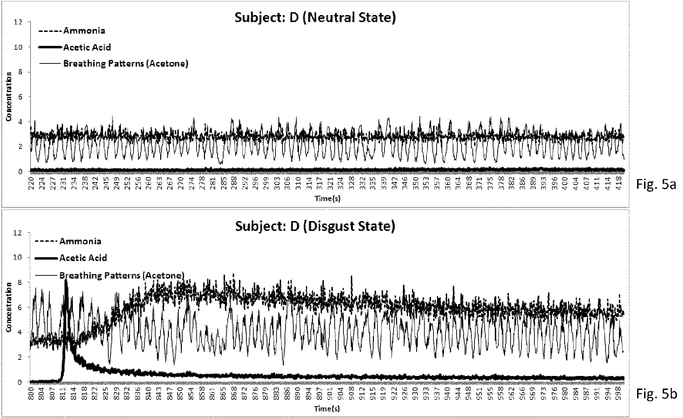

The same pattern is seen in Figs. 5a and 5b, showing the correlation between ammonia and acetic acid together with acetone in another example individual D in a neutral state and a state of disgust, respectively.

Figs 4 and 5 only show the measurements for two individuals, the same pattern has been found and confirmed in numerous other measurements on the same and other individuals.

It has been concluded that disgust can be determined based on measurement of these molecules, by measuring the quantities of either or both of these molecules, in absolute terms, by measuring the quantities in relation to a calibration value, such as the level of acetone, or by trend analysis over time, etc.

It has also been found that when negative emotion(s) is in full swing, no significant amount of "happy" molecular (NON-propanol) fragments could be observed, and vice versa (this is however not illustrated in the drawings, since further black-and-white graphs in the same plot would ruin the visibility).

### Conclusion and summary

The invention has now been discussed in relation to different embodiments. However, it should be appreciated by those versed in the art that several further alternatives are possible. For example, the features of the different embodiments discussed above may naturally be combined in many other ways. For example, feedback or output of the identified mental state(s) may be communicated in many other ways than the ones discussed above. Further, various parts of the apparatus and system may be arranged together or separated from each other etc.

It is further possible to use the invention for identification of many other mental states than the ones exemplified above.

Further, the method and apparatus is very useful also in many other applications and fields.

It will be appreciated by those versed in the art that several such alternatives similar to those described above could be used without departing from the spirit of the invention, and all such modifications should be regarded as a part of the present invention, as defined in the appended claims.

## Claims

1. A method for automated identification of at least one transitory emotional state of a living mammal, comprising:
mounting at least one sensor on or in the vicinity of said living mammal, said sensor being configured to detect at least one metabolite molecule(s), and preferably volatile organic compound(s) or volatile metabolic molecule(s), emanated by the living mammal;
determining continuously and in real-time the amount of at least one of said metabolite molecule(s) with said sensor over a period of time;
analyzing said determined amount of said at least one metabolite molecule(s) to establish whether the amount or the increase rate of said at least one metabolite molecule(s) exceeds at least one predetermined criterion, and when such a predetermined criterion has been exceeded, identifying that a transitory emotional state associated with said at least one metabolite molecule(s) is present in the living mammal.

2. The method of claim 1, wherein the transitory emotional state is at least one of fear, stress, anger, sadness, disgust, surprise, laughter and happiness.

3. The method of claim 1 or 2, wherein the at least one sensor comprises a sensor configured to be mounted in or in the vicinity of a mouth or nose of the living mammal, said sensor being configured to detect at least one metabolite molecule(s) emitted by the living mammal in its breath.

4. The method of claim 3, wherein the time period for determining continuously the amount of at least one of said metabolite molecule(s) extends over at least one, and preferably a plurality, of breathing cycles.

5. The method of any one of the preceding claims, wherein the at least one sensor comprises a sensor configured to be mounted on the skin of the living mammal, said sensor being configured to detect at least one metabolite molecule(s) emitted through the skin of the living mammal.

6. The method of any one of the preceding claims, wherein the at least one metabolite molecule(s) comprises at least one of the molecule(s): ammonia, acetic acid, acrolein or molecules with molecular formula C₃H₄O, and a molecule that produce ion fragments C₃H₇⁺ and C₃H₅⁺.

7. The method of any one of the preceding claims, wherein the at least one predetermined criterion is based on at least one of: a normal level for said at least one metabolite molecule(s) established by previous measurements on the living mammal; and a normal level for said at least one metabolite molecule(s) established by previous measurements on other living mammal.

8. The method of any one of the preceding claims. further comprising the step of using the identified transitional emotional state in at least one of overcoming communication difficulties and help in communication;
monitoring post surgery recovery;
monitoring sleep quality;
monitoring pattern in sleep disorder;
testing of commercial audio and/or video media;
reliability testing of suspects;
reliability testing on accused and witness during judicial trials;
self performance monitoring;
professional performance monitoring;
customizing mobile content delivery;
customizing environment changing;
customizing robotic response;
customizing learning and memory training environment design in real time;
monitoring progress of psychological therapy;
diagnosing mental disease;
diagnosing state of dementia;
diagnosing depression;
quality ensuring consumer research;
testing during neuromarketing;
emotional status tracking in vulnerable subject, e.g. during bullying;
emotional tracking of patients under anesthesia or coma;
promoting physical training;
aiding emotional communication, in particular with patients suffering from dementia, mental retardness, stroke and brain damage, and with animals; and
aiding in Human Resource selection.

9. The method of any one of the preceding claims, for identification of laughter and happiness, and wherein the metabolite molecule(s) are one or several molecules producing ion fragments of C₃H₇⁺ and/or C₃H₅⁺.

10. An apparatus for automated identification of at least one transitory emotional state of a living mammal, comprising:
at least one sensor configured to be mounted on or in the vicinity of said living mammal, said sensor being configured to detect in real-time at least one metabolite molecule(s), and preferably at least one volatile organic compound(s), emitted by the living mammal;
a memory to store data related to the amount of at least one of said metabolite molecule(s) during continuous measurement with said sensor over a period of time;
an analyzer programmed to analyze said determined amount of said at least one metabolite molecule(s) to establish whether the amount or the increase rate of said at least one metabolite molecule(s) exceeds at least one predetermined criterion, and when such a predetermined criterion has been exceeded, identifying that a transitory emotional state associated with said at least one metabolite molecule(s) is present in the living mammal.

11. The apparatus of claim 10, wherein the sensor is arranged remote from said analyzer, the analyzer and the sensor being connected by a wired or wireless connection.

12. The apparatus of claim 10 or 11, further comprising a feedback provider providing a visual or audial indication of the identified transitory emotional state.

13. The apparatus of any one of the claims 10-12, wherein the at least one sensor comprises a sensor configured to be mounted in or in the vicinity of a mouth or nose of the living mammal, said sensor being configured to detect at least one metabolite molecule(s) emitted by the living mammal in its breath, said sensor preferably being attachable to a nostril of the living mammal, and e.g. comprising a clip-on sensor comprising a pair of jaws to clip the module partially within said nostril.

14. The apparatus of any one of the claims 10-13, further comprising at least one of: an output device, such as a display, an alarm, or the like; an interface for communicating data, such as measurement data and/or data about determined transitory emotional state(s) to an external device, and preferably through wireless communication; and an input device to provide control data, setting data, reconfiguration data or the like to the apparatus.

15. Use of one or several of C₃H₇⁺ and C₃H₅⁺ as a biomarker for identification of the transitory emotional state of happiness.
